# EUROPEAN PATENT SPECIFICATION

(11) **EP 3 266 487 B1**
(45) Date of publication and mention of the grant of the patent: **12.10.2022**
(21) Application number: 16761527.7
(22) Date of filing: 26.02.2016
(51) Int. Cl.: A61M 25/01, A61B 1/00, A61M 25/10, A61M 25/00, A61B 17/22

(54) **TREATMENT INSTRUMENT FOR ENDOSCOPE**
BEHANDLUNGSINSTRUMENT FÜR ENDOSKOP
INSTRUMENT DE TRAITEMENT POUR ENDOSCOPE

(30) Priority: 06.03.2015 JP 2015045032
(43) Date of publication of application: 10.01.2018
(73) Proprietor: Zeon Corporation, Tokyo 100-8246 (JP)
(72) Inventor: KAWAJIRI, Koji, Tokyo 100-8246 (JP)
(74) Representative: Maiwald Patent- und Rechtsanwaltsgesellschaft mbH
(86) International application number: PCT/JP2016/055800
(87) International publication number: WO 2016/143556

(56) References cited:
- WO-A1-2012/042619
- GB-A- 2 471 517
- JP-A- 2009 526 616
- JP-A- 2010 504 837
- US-A- 5 413 559
- US-A1- 2007 021 771
- US-A1- 2007 191 767
- US-A1- 2008 221 550
- US-B1- 6 605 062
- US-B1- 7 195 611

## Description

### TECHNICAL FIELD

The present invention relates to a treatment instrument for an endoscope such as balloon catheter for removing calculus which is formed in bile duct or so.

### BACKGROUND ART

Several methods are known as the method for removing the calculus which is formed in the bile duct, that is the gall stone, by taking out from the body; and the method of using the balloon catheter is known as one of them. When removing the gall stone from the bile duct using the balloon catheter, first the balloon catheter is inserted in the bile duct via the endoscope while the balloon is deflated, then the balloon is positioned deeper than the position of the gall stone to be removed. Next, the balloon is inflated, and then the balloon catheter is pulled back so that the gall stone is scraped by the balloon, thereby the gall stone is discharged out of the bile duct.

As the balloon catheter for removing the calculus such as for removing the gall stone as discussed in above, for example those having the structure shown in Patent document 1 and Patent document 2 are known. The balloon catheters described in these documents have the balloon made of an elastic material bonded to the tip part (the distal end part) of the catheter tube, and the fluid is introduced in this balloon, thereby the balloon is inflated.

In order to move the distal end part of the conventional balloon catheter for removing the calculus equipped with such balloon to the predetermined position of goal in the bile duct, a guide wire is used. That is, the guide wire is inserted in the body of the patient using the endoscope, then the distal end part of the guide wire is positioned to the predetermined position in the bile duct, then along said guide wire, the balloon catheter is moved. Therefore, at the balloon catheter, a wire lumen for passing the guide wire is formed along the longitudinal direction of said catheter tube.

However, for the conventional balloon catheter for removing the calculus, the wire lumen was formed along the entire length of the catheter tube, thus a process of inserting the balloon catheter from the proximal end side of the guide wire and a process of removing the balloon catheter were not easy and these processes were complicated. That is, in order to move the balloon catheter provided along the guide wire in relative to the guide wire, it is necessary to hold both the balloon catheter and the guide wire, thus the proximal end side of the guide wire at least needs to be longer than the entire length of the catheter tube so that the guide wire is projected out from the endoscope; and it is complicated to control such.

Note that, a opening connecting to the wire lumen is provided in the middle of the entire length of catheter tube, and the guide wire may be enter and exit through there. However, in such structure, it is difficult to pull out the proximal end of the guide wire from an opening. Also, the tube will become weak in case an opening is simply provided to the catheter tube, and the catheter tube easily kinks from that opening. Such balloon catheters are for example disclosed in WO2012/04619 and WO9320882.

### PRIOR ART DOCUMENT

Patent document 1: JP Utility Model Application Laid Open No.H05-63551
Patent document 2: JP Patent Application Laid Open No.

### SUMMARY OF THE INVENTION

### TECHNICAL PROBLEMS TO BE SOLVED BY THE INVENTION

The present invention is attained in view of such problems, and the object is to provide the treatment instrument for endoscope capable of easy insertion of the treatment instrument for endoscope from the proximal end side of the guide wire, and easy removal of the treatment instrument for endoscope, also having excellent handling property, and capable of easy exchange for other treatment instrument for the endoscope.

### MEANS FOR SOLVING THE PROBLEMS

In order to attain the above object, the treatment instrument for the endoscope according to the present invention comprises a multi-lumen tube formed with multiple lumens including a main lumen along longitudinal direction, and a treating part provided at a distal end part of said multi-lumen tube, wherein
a distal side wire insertion opening connecting to said main lumen is formed at the distal end of said multi-lumen tube,
a proximal side wire insertion opening connecting to said main lumen is formed in a middle of the longitudinal direction of said multi-lumen tube at a proximal side than said treating part,
a tube member is inserted towards the distal end direction of said main lumen from said proximal side wire insertion opening, and
a peripheral of a proximal end opening of said tube member is bonded to a peripheral of said proximal side wire insertion opening and, an outer peripheral face of the distal end opening of said tube member is adhered to the inner circumference face of said main lumen.

For the treatment instrument for the endoscope according to the present invention, a proximal side wire insertion opening connecting to the main lumen is formed at the position in the middle of the longitudinal direction of the multi-lumen tube at further the proximal side than the treating part. Therefore, the guide wire of the proximal end side can protrude out from the endoscope in a length slightly longer than the length between the distal side wire insertion opening of the catheter tube to the proximal side wire insertion opening.

As a result, the process of inserting the treatment instrument for the endoscope from the proximal end side of the guide wire, and the process of removing the treatment instrument of the endoscope can be done easily, hence the handling property improves and the exchange of the treatment instrument for the endoscope can be done easily as well. Further, the length of the guide wire to be protruding out from the endoscope can be shortened, thus the sanitary management thereof becomes easy.

For the treatment instrument for the endoscope according to the present invention, the tube member is inserted from the proximal side wire insertion opening towards the distal end direction of the main lumen, thus just by pushing the proximal end of the guide wire from the distal side wire insertion opening, the proximal end of the guide wire is guided towards the proximal side wire insertion opening through the tube member, and the guide wire can be easily pulled therefrom. Also, the tube member is inserted from the proximal side wire insertion opening towards the distal end direction of the main lumen, therefore a part close to the proximal side wire insertion opening is reinforced by the tube member, and the multi-lumen tube can be effectively prevented from kinking.

At said main lumen positioned at further proximal side than the proximal end of said tube member, a stylet can be inserted in a removable manner. By inserting the stylet, the rigidity of the proximal end part of the multi-lumen tube is enhanced; hence the insertion characteristic of the treatment instrument for the endoscope along the guide wire can be improved as well.

At the proximal end of said tube member, a flange part is integrally formed, and said flange part may be bonded to the outer peripheral face of said multi-lumen tube positioned around said proximal side wire insertion opening. By constituting as such, the periphery of the proximal end opening of the tube member can be easily bonded to the periphery of the proximal side wire insertion opening.

At said treating part, the balloon is installed to the distal end part of said multi-lumen tube, and the inside of said balloon part is connected to one of balloon lumen among the lumen of said multi-lumen tube. According to the treatment instrument for the endoscope having such constitution, the calculus such as gall stone or so can be easily discharged out of the body rapidly using the balloon.

Preferably, the length of said tube member is the length wherein the distal end of said tube member does not protrude out from said distal side wire insertion opening; however it may protrude out somewhat. By setting the length of the tube member to the length so that the distal end of the tube member does not protrude out from the distal side wire insertion opening, the length of the tube member can be shortened, and the production becomes easy. Also, if the tube member can be shortened, then only the surrounding area of the proximal side wire insertion opening can be reinforced, and also the insertion characteristic of the treatment instrument for endoscope into the body can be improved without compromising the resilience at the distal end part of the multi-lumen tube.

Preferably, at the distal end part of said multi-lumen tube, a small diameter part is formed wherein the outer diameter is smaller than other part of said multi-lumen tube, and said small diameter part is equipped with said treating part.

According to such constitution, in the lumen of the multi-lumen tube having relatively large diameter, the tube member is housed; and the tube member can be easily inserted, also the resilience at the distal end part of the multi-lumen tube is not compromised, and the insertion characteristic of the treatment instrument for the endoscope into the body is improved.

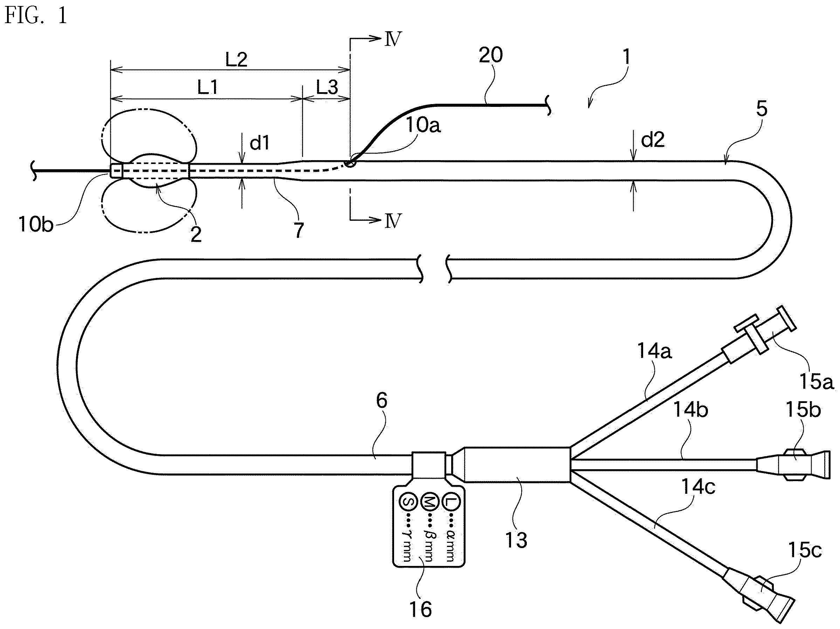

[ig.1] Fig.1 is an entire figure of the balloon catheter for removing the calculus according to one embodiment of the present invention.
[Fig.2] Fig.2 is a partial enlarged view of the distal end part of the catheter tube of the balloon catheter for removing the calculus shown in Fig.1.
[Fig.3] Fig.3 is an enlarged cross section along III-III line shown in Fig.2.
[Fig.4] Fig.4 is an enlarged cross section along IV-IV line shown in Fig.1.
[Fig.5A] Fig.5A is a partial cross section view showing how to make the proximal side wire insertion opening in the catheter tube shown in Fig.1.
[Fig.5B] Fig.5B is a partial cross section showing the step following Fig.5A.
[Fig.5C] Fig.5C is a partial cross section showing the step following Fig.5B.
[Fig.5D] Fig.5D is a partial cross section showing the step following Fig.5C.
[Fig.5E] Fig.5E is a partial cross section showing the step following Fig.5D.
[Fig.6(A)] Fig.6(A) is a perspective view of the tube member according to other embodiment of the present invention.
[Fig.6(B)] Fig.6(B) is a partial cross section showing the example of use of the tube member thereof.
[Fig.7(A)] Fig.7(A) is a perspective view of the tube member according to other embodiment of the present invention.
[Fig.7(B)] Fig.7(B) is a partial cross section showing the example of use of the tube member thereof.
[Fig.8(A)] Fig.8(A) is a perspective view of the tube member according to other embodiment of the present invention.
[Fig.8(B)] Fig.8(B) is a partial cross section showing the example of use of the tube member thereof.
[Fig.9] Fig.9 is a partial enlarged view showing the surrounding of the proximal side wire insertion opening of the catheter tube where the tube member shown in Fig.8(A) is installed.

### EFFECT OF THE PRESENT INVENTION

Hereinafter, the present invention will be described based on the embodiment shown in Fig. 1. The invention is defined in claim 1. Several embodiments are defined in the dependent claims.

### First Embodiment

As shown in Fig.1, the balloon catheter 1 for removing calculus as the treatment instrument for endoscope according to one embodiment of the present invention comprises a catheter tube 5, a balloon (treating part) 2, a cover 13, three branch tubes 14a to 14c, and three hubs 15a to 15c.

The catheter tube 5 of the balloon catheter 1 for removing the calculus is a tube which is formed by a flexible material, and comprises a distal end part 7 which is the end part endoscopically inserted into the body, and a proximal end part 6 positioned at other end side thereof. The outer diameter 2 of the proximal end part 6 of this catheter tube 5 is usually 1.0 to 4.2 mm, and the entire length is usually 500 to 2500 mm. Also, the material of the catheter tube 5 is not particularly limited as long as it has elasticity, but preferably it is a polymer material, and polyamide resin or polyamide based elastomer are particularly preferable.

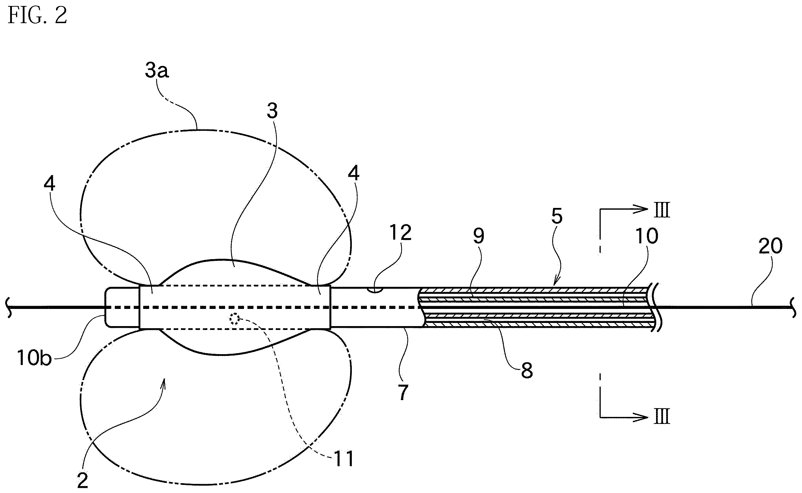

As shown in Fig.2, at the inside of the catheter tube 5, the balloon lumen 8, a contrast agent lumen 9, and a main lumen 10 are formed. That is, the catheter tube 5 constitutes a multi-lumen tube. The balloon lumen 8 is a lumen which is the flow passage for sending the fluid into the balloon 2 such as air for inflating the balloon 2; and it runs through from the proximal end of the catheter tube 5 to a fluid discharge opening 11 provided at the distal end part 7 of the catheter tube 5 so that it is positioned inside the balloon 2.

The contrast agent lumen 9 is the lumen used as the flow passage of the contrast agent in case of taking X ray contrast to identify the position of the calculus. This contrast agent lumen 9 runs through from the proximal end of the catheter tube 5 to the exhaustion opening 12 of the distal end part 7 of the catheter tube 5. The exhaustion opening 12 is an opening provided at the outside of the balloon 2, preferably provided to the position further proximal than the balloon 2.

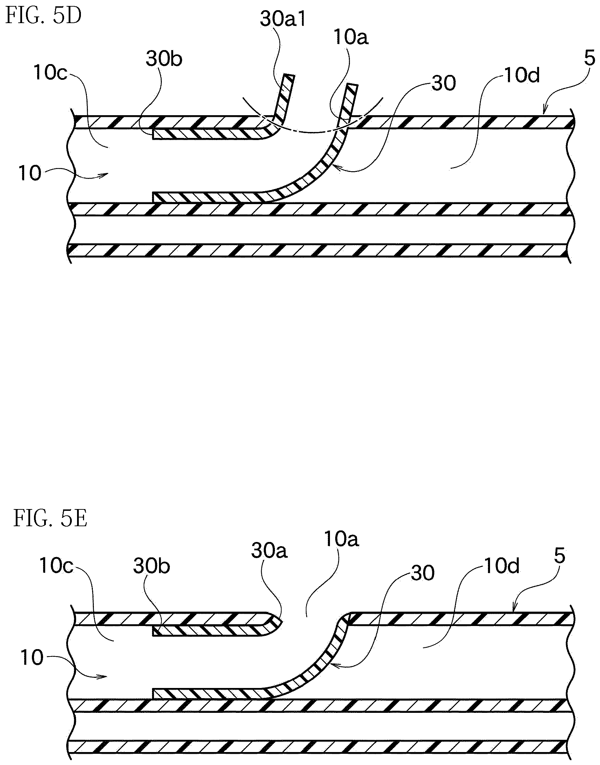

The main lumen 10 runs through from the proximal end to the distal end of the catheter tube 5; but it has different function at the distal side and the proximal side across the proximal side wire insertion opening 10a shown in Fig.1. That is, the distal end opening of the main lumen 10 is formed at the distal end of the tube 5 positioned at the distal side of the balloon 2 as the distal side wire insertion opening 10b, and the distal side main lumen 10c positioned between the proximal side wire insertion opening 10a and the distal side wire insertion opening 10b functions as the wire lumen. Also, the proximal side main lumen 10d of the main lumen 10 positioned at the proximal side than the proximal side wire insertion opening 10a shown in Fig.5E functions for example as the lumen for inserting the stylet or so. Note that, the stylet increases the rigidity of the catheter tube 5, and thus it is used to improve the insertion property of the balloon catheter 1 for removing the calculus with respect to the endoscope and the body. For example stylet is a filament form body (twisted or straight or so) or a rod form body constituted by a metal such as stainless steel or so.

As shown in Fig.1, the proximal side wire insertion opening 10a is formed at the position having an outer diameter d2 which is the position in the middle along the longitudinal direction of the catheter tube 5 at further proximal side than balloon 2, and the position further proximal side than the distal end part 7 having small diameter. The length L2 between the proximal side wire insertion opening 10a and the distal side wire insertion opening 10b is longer than the longitudinal direction length L1 of the distal end part 7 having a small diameter, and it is preferably 35 to 800 mm. Also, the length L3 = L2 - L1 is preferably 5 to 400 mm.

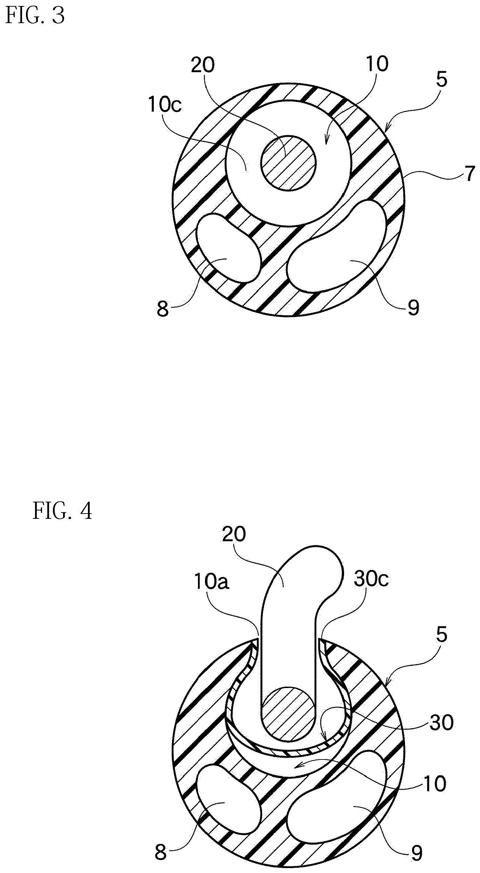

As shown in Fig.4 and Fig.5E, the periphery of the proximal side wire insertion opening 10a is bonded with the periphery of the proximal end opening 30a having a circular cross section of the tube member 30, and the distal end opening 30b of the tube member 30 is inserted from the proximal side wire insertion opening 10a towards the distal end direction of the main lumen 10. The length of the tube member 30 is preferably about the length wherein the distal end of the tube member 30 does not protrude out from the distal side wire insertion opening 10b shown in Fig.1.

Further, the length of the tube member 30 is preferably equal or less than the length L3 shown in Fig.1. At the position of the length L1 shown in Fig.1, the inner diameter of the main lumen 10 of the catheter tube 5 shown in Fig.3 becomes smaller, accordingly the outer diameter of the distal end part of the tube member 30 needs to be smaller. Further, the outer peripheral face of the distal end opening 30b of the tube member 30 is preferably adhered to the inner circumference face of the distal side main lumen 10c; and the opening peripheral part of the distal end opening 30b preferably has an inner circumference face of a taper form wherein the thickness of the tube member 30 becomes thinner towards the distal side so that the proximal end of the wire 20 does not get caught.

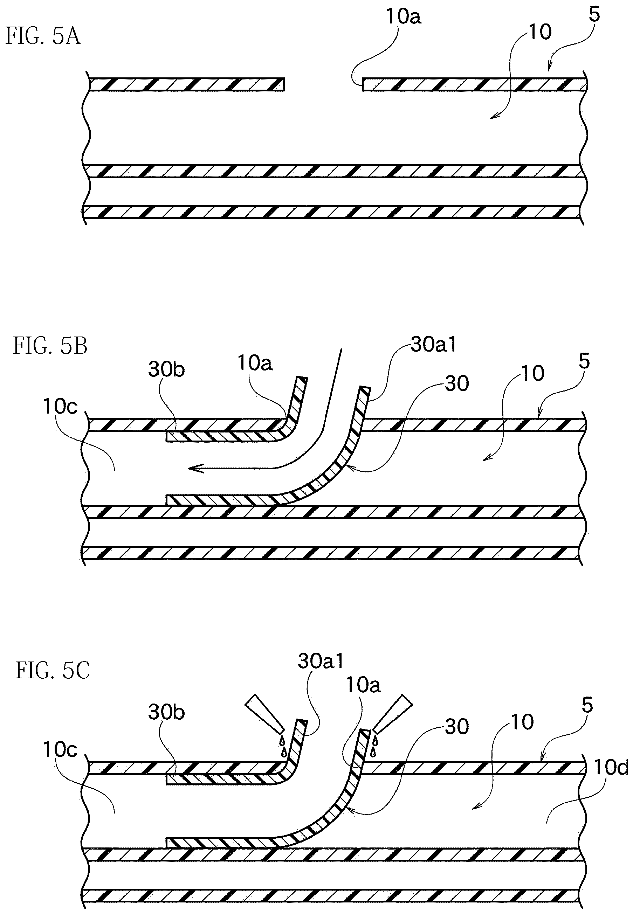

In order to attain such structure, for example, first as shown in Fig.5A, the proximal side wire insertion opening 10a connecting only to the main lumen 10 is formed at the predetermined position (the position of L2 shown in Fig.1) in the longitudinal direction of the catheter tube 5. The opening of the insertion opening 10a is circular, and the inner diameter thereof is about the same as the outer diameter of the tube member 30 shown in Fig.5B, further it is about the size that the tube member 30 can enter while closely attaching to the inside of the insertion opening 10a.

In the present embodiment, the tube member 30 is constituted by a short tube having excellent flexibility with uniform outer diameter and inner diameter along the longitudinal direction, and for example the tube member 30 is constituted by a material such as polyamide resin, polyamide based elastomer, polyolefin based resin, polyvinyl chloride resin or so. Further, the tube member 30 is constituted by the material having same or even more excellent elasticity than the catheter tube 5. This is to maintain the flexibility at the distal end part of the catheter tube 5.

At the inside of the tube member 30, the guide wire 20 shown in Fig.1 and Fig.2 is passed through, thus at least the inner peripheral face of the tube member 30 is preferably constituted by the material having excellent slip characteristic. Also, the thickness of the tube member 30 is not particularly limited, and it may be about the thickness which is not damaged by the movement of the guide wire 20, and as long as the strength is maintained, the thinner it is, the better it is.

As shown in Fig.5B, after the tube member 30 is inserted inside the proximal side wire insertion opening 10a so that the distal end opening 30b of the tube member 30 is positioned at the distal side main lumen 10c of the main lumen, the adhesive agent is introduced to the contact part between the periphery of the insertion opening 10a, at the surrounding of the proximal end opening part 30a1 of the tube member 30 which is protruding out from the proximal side wire insertion opening 10a as shown in Fig.5C.

Then, the adhesive agent is cured, and as shown in Fig.5D, the proximal end opening part 30a1 which is the excess part of the tube member 30 protruding out from the proximal side wire insertion opening 10a is cut off. As a result, as shown in Fig.5E, the structure wherein the proximal end opening 30a of the tube member 30 is smoothly bonded to the proximal side wire insertion opening 10a of the catheter tube 5 is obtained, and the tube member 30 is prevented from protruding out from the insertion opening 10a.

The cross section shape of the balloon lumen 8, the contrast agent lumen 9, and the main lumen 10 shown in Fig.3 and Fig.4 are not particularly limited, and these may be a shape which can be efficiently placed in the catheter tube respectively. Note that, for the main lumen 10, preferably the cross section shape is circle as similar to the cross section of the general guide wire 20. Also, the cross section area of the balloon lumen 8 is preferably 0.03 to 0.5 mm², the cross section area of the contrast agent lumen 9 is preferably 0.08 to 1.0 mm², and the cross section area of the main lumen 10 is preferably 0.5 to 2.0 mm².

The balloon 2 of the balloon catheter 1 for removing the calculus is installed at the distal end part of the catheter tube 5 so as to cover the fluid discharge opening 11. This balloon 2 is formed by an elastic material, and by introducing the fluid to the inside via the balloon lumen 8 of the catheter tube 5, the balloon 2 is inflated. Due to this inflated balloon 2, the calculus in the body can be removed by scraping out or pushing out.

As the elastic material forming the balloon 2, the material having 0.1 to 10 MPa of 100% modulus (the value in accordance with JIS K 6251) is preferable, and 1 to 5 MPa is particularly preferable. If the 100% modulus is too large, the strength of the balloon 2 may become insufficient, and if too large, the balloon 2 may not inflate to a sufficient size. As the specific example of suitable elastic material for forming the balloon 2, natural rubber, silicone rubber, polyurethane elastomer or so may be mentioned.

As shown in Fig.2, the balloon 2 is a tube form as a whole, and at the both end part thereof, the bonding part 4 bonding with the outer peripheral face of the catheter tube 5 is formed. In between the bonding part 4 at the both ends thereof, the inflating part 3 which inflates by introducing the fluid to the inside is formed (in Fig.2, the inflating part not receiving the external force is shown in bold line, and the inflating part 3a inflated by introducing the fluid is shown in two-dot chain line).

Under the condition of not receiving the external force, the inflating part 3 of this balloon 2 is formed to have a rotary body shape wherein the convex curved line is rotated towards the outside of the catheter tube 5 taking the center axis of the catheter tube 5 as the rotating axis. That is, the inflating part 3 of the balloon 2 installed to the catheter tube 5 has such rotary body shape as mentioned in above, when the pressure at the inside and the outside is balanced (the condition wherein the fluid for inflating the balloon 2 is not introduced into the inside of the balloon 2).

As the inflating part 3 of the balloon 2 forms such shape, compared to the conventional balloon having a circular cylinder shape when the external force is not applied, the balloon 2 can be inflated to have large outer diameter. Note that, the curved line as the generating line of the rotary body shape is not particularly limited as long as it is a convex curved line towards the outside of the catheter tube 5. It may be any curved line, but preferably it is the curved line on the same plane as the center axis of the catheter tube 5.

The inflating part 3 of the balloon 2 while not receiving the external force preferably has the maximum outer diameter which is 110 to 200% of the minimum outer diameter. Also, the length of the inflating part 3 of the balloon 2 (the length along the longitudinal direction of the catheter tube 5) is preferably 5 to 20 mm, and the thickness is preferably 0.10 to 0.50 mm.

For the balloon catheter 1 for removing the calculus, the exhaustion opening for discharging the contrast agent is preferably provided to the surface of the catheter tube 5 at the position within 10 mm (more preferably within 5 mm) to the proximal side from the proximal end of the balloon 2. By providing the exhaustion opening 12 at this position, the body lumen is sealed by inflating the balloon 2 then the contrast agent is discharged from the exhaustion opening 12, thereby the body lumen positioned at the proximal side than the inflated balloon 2 can be contrasted.

The shape of the bonding part 4 positioned at the both end sides of the inflating part 3 of the balloon 2 is not particularly limited as long as it is a shape capable of bonding with the distal end part 7 of the catheter tube 5; but preferably it has circular cylinder shape. In case the bonding part 4 of the balloon 2 is a circular cylinder shape, the inner diameter thereof is preferably about the same as the outer diameter of the catheter tube 5, and the length is preferably 0.5 to 5 mm. Also, the thickness of the bonding part 4 of the balloon 2 is not particularly limited, and for example it may be substantially the same as the inflating part 3. Note that, the method of bonding the bonding part 4 of the balloon 2 and the distal end part 7 of the catheter tube 5 is not particularly limited, and for example the adhesion using an adhesive agent, a heat sealing, welding using a solvent, and ultrasonic welding or so may be mentioned.

Not forming part of the invention, the method for producing the balloon 2 having the above mentioned shape is not particularly limited, and the known method as the membrane production of the elastic material may be used, but dipping method is preferably used. For the dipping method, the elastic material and various additives depending on needs are dissolved in the solvent to form a solution or suspension, then the mold having the outer shape almost the same as the shape of the desired balloon is immersed in this solution (suspension) to coat the surface of the mold by the solution (suspension). Then, the solvent is evaporated and the coating film is formed to the surface of the mold. By repeating this immersing and drying, the balloon having desired thickness can be produced. Note that, depending on the type of the elastic material, if necessary, the crosslinking is carried out after producing the film.

For the balloon catheter 1 for removing the calculus, as the present embodiment shown in Fig.1, the distal end part 7 of the catheter tube 5 where the balloon 2 is installed preferably has the small diameter part wherein the outer diameter is smaller than the other part (the proximal end part 6) of the catheter tube 5. The balloon catheter tube 1 for removing the calculus can inflate the balloon sufficiently large even if the part of the catheter tube 5 (the distal end part 7) where the balloon 2 is installed has a small diameter, and also while maintaining the rigidity of the proximal end part 6 of the catheter tube 5 to some degree, and by giving flexibility to the distal end part 7 by having small diameter, the handling property of the balloon catheter 1 for removing the calculus can be improved. In this case, the outer diameter d1 of the distal end part 7 of the catheter tube 5 is preferably 50 to 95%, and particularly preferably 60 to 90% of the outer diameter d2 of the proximal end part 6.

The method for making the distal end part 7 of the catheter tube 5 to have smaller diameter than the proximal end part 6 is not particularly limited, but preferably the catheter tube 5 at the boundary between the distal end part 7 and the proximal end part 6 is preferably formed into taper shape so that it is smaller towards the distal end. Also, as other method for making the distal end part 7 smaller than the proximal end part 6, the step may be provided between the distal end part 7 and the proximal end part 6. The length L1 in the longitudinal direction of the distal end part 7 having small diameter is preferably 30 to 400 mm.

The branch pipes 14a to 14c of the balloon catheter 1 for removing the calculus are the tubes connected to each lumen so that the procedure of sending the fluid to the balloon lumen 8 of the catheter tube 5, the procedure of introducing the contrast agent to the contrast agent lumen 9, or the procedure of inserting the stylet to the proximal end side of the main lumen 10 can be done easily.

The material of the branch pipes 14a to 14c is not particularly limited, but preferably the polymer material may be used. Also, the connecting method of branch pipes 14a to 14c with each lumen of the catheter tube 5 is not particularly limited, but the distal end part of the branch pipes 14a to 14c are formed into a taper shape, and the adhesive agent is coated around the outer peripheral face thereof, then the end part is inserted into the lumen of the catheter tube 5, thereby the adhesion may be carried out.

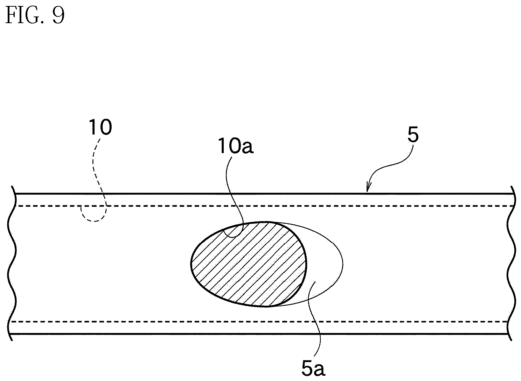

The hubs 15a to 15c of the balloon catheter 1 for removing the calculus is the member connected to the proximal end side of the branch pipes 14a to 14c. For example the hub 15a and the branch pipe 14a are connected to the balloon lumen 8 shown in Fig.2, and the balloon expansion fluid can be introduced or drawn out from the hub 15a. Also, the hub 15c and the branch pipe 14c are connected to the contrast agent lumen 9 shown in Fig.9, and the contrast fluid can be introduced or drawn out from the hub 15c. The hub 15b and the branch pipe 14b are connected to the proximal side main lumen 10d shown in Fig.5E, and the stylet can be introduced or drawn out from the hub 15b. The material of hubs 15a to 15c is not particularly limited, but preferably a transparent polymer material is used.

The cover 13 of the balloon catheter 1 for removing the calculus shown in Fig.1 is provided to reinforce and protect the connecting part between the catheter tube 5 and the branch pipes 14a to 14c, thus it is provided so as to cover the connecting part. The shape of the cover 13 is not particularly limited, but usually it is a box shape or circular cylindrical shape. The material of the cover 13 is not particularly limited, but preferably the polymer material is used. Also, a heat shrinkable tube can be used as the cover 13.

At the distal end side of the cover 13, the tag 16 is installed to the outer periphery of the catheter tube 5. The tag 16 shows, for example, specific information about the particular balloon catheter 1 such as how much the outer diameter of the balloon 2 expands by how much air amount or so.

Next, not forming part of the invention, as the example of use of the balloon catheter 1 for removing the calculus of the present disclosure, the example of removing the gall stone from bile duct will be explained.

First, the endoscope is inserted into the body, and then the tip of the endoscope is positioned near the entrance of the bile duct (duodenal papilla). Next, by using a cannulation catheter or so if needed, the guide wire 20 is inserted into the patient's body via the channel of the endoscope, and the distal end of the guide wire 20 is guided into the biliary duct. Here, the guide wire 20 having appropriate length is used so that proximal end side part of the guide wire 20 protrudes out from the endoscope by a length slightly longer than the length (length L2) between the proximal side wire insertion opening 10a and the distal end side wire insertion opening 10b. Next, if needed, the stylet is inserted into the proximal side main lumen 10d via the hub 15b and the branch pipe 14b, and also the guide wire 20 is passed to the distal side main lumen (wire lumen) 10c between the proximal side wire insertion opening 10a and the distal side wire insertion opening 10b from the distal side wire insertion opening 10b side. Then, while the balloon 2 is not inflated, from the distal end side of the catheter tube 5, the balloon catheter tube 1 for removing the calculus is inserted into the body along the guide wire 20 via the channel of the endoscope, and the distal end part of the catheter 1 is guided into the bile duct.

Next, the catheter 1 is pushed deeper into the bile duct, then air is sent into the balloon 2 via the hub 15a, the branch pipe 14a and the balloon lumen 8 using syringe or so, thereby the balloon 2 is inflated.

Next, the contrast agent is introduced to the exhaustion opening 12 via the hub 15c, the branch pipe 14c and the contrast agent lumen 9 using syringe or so, then the contrast agent is discharged and the X ray contrast of the inside of the bile duct is taken, thereby the condition of the gall stone is verified. Next, while the balloon 2 is kept inflated, the catheter 1 is pulled back, and then the gall stone can be scraped out from the duodenal papilla to the outside of the bile duct by the balloon 2.

Here, the catheter 1 of the present embodiment can inflate the balloon 2 to a sufficient size, thus a space is rarely formed between the inner wall of the bile duct and the balloon 2, and the gall stone can be easily taken out. Note that, usually the gall stone scraped out of the bile duct is naturally excreted from the body. That is, according to the balloon catheter 1 of the present embodiment, the calculus such as the gall stone or so can be easily excreted out of the body rapidly using the balloon 2.

Also, when it is necessary to exchange the balloon catheter 1 with other treatment instrument for endoscope such as other balloon catheter or so, while leaving the distal end of the guide wire 20 in the body, only the balloon catheter 1 is pulled out of the body along the guide wire 20. Here, the guide wire 20 is passed through the distal side main lumen 10c by a relatively short length which is from the proximal side wire insertion opening 10a positioned in the middle of the catheter tube 5 to the distal side wire insertion opening 10b, thus the catheter 1 can be easily taken out.

That is, the proximal end side of the guide wire 20 may be pulled out from the endoscope by a length slightly longer at least than the length between the distal side wire insertion opening 10b to the proximal side wire insertion opening 10a of the catheter tube 5. As a result, not only the process of inserting the guide wire 20 to the distal side main lumen 10c, but also the process of removing the balloon catheter 1 along the guide wire 20 can be done easily. Thus, the handling property improves. Also, the balloon catheter 1 can be easily exchanged to other treatment instrument for endoscope. Further, the length of the guide wire 20 being pulled out of the endoscope can be shortened, and the sanitary management is easy.

For the balloon catheter 1 of the present embodiment, the tube member 30 is inserted from the proximal side wire insertion opening 10a to the distal end direction of the main lumen 10, hence just by pushing the proximal end of the guide wire 20 from the distal side wire insertion opening 10b, the proximal end of the guide wire 20 is guided to the proximal side wire insertion opening 10a via the tube member 30, then the guide wire 20 is pulled out therefrom. Hence, the balloon catheter 1 of the present embodiment allows easy insertion of the guide wire 20 which is pulled out from the endoscope compared to the balloon catheter without the tube member 30 and a simple side opening is used as the proximal side wire insertion opening.

Also, since the tube member 30 is inserted from the proximal side wire insertion opening 10a to the distal end direction of the main lumen 10, the area near the proximal side wire insertion opening 10a is reinforced by the tube member 30, thus the catheter tube 5 made of multi-lumen tube can be effectively prevented from kinking.

Further, at the proximal side main lumen 10d positioned at further proximal side than the proximal end of the tube member 30, the stylet is inserted in a removable manner, thus the rigidity of the proximal end part of the catheter tube 5 increases, and the insertion characteristicinsertion characteristics of the balloon catheter 1 along the guide wire 20 is improved.

Also, the length of the tube member 30 is a length wherein the distal end of the tube member 30 does not protrude out from the distal side wire insertion opening 10b, but it may slightly protrude out therefrom. The length of the tube member 30 is a length wherein the distal end of the tube member 30 does not protrude out from the distal side wire insertion opening 10b, thereby the length of the tube member 30 can be shortened, and the production becomes easy. Also, in case the length of the tube member 30 is shortened, the area near the proximal side wire insertion opening 10a can be reinforced, and also the insertion characteristic of the balloon catheter 1 into the body can be improved without compromising the flexibility of the distal end part 7 of the catheter tube 5.

Further, in the present embodiment, as shown in Fig.1, the tube member 30 is housed in the distal side main lumen 10c of the catheter tube 5 having relatively large diameter d2, the tube member 30 can be easily inserted, and also the insertion characteristic of the balloon catheter 1 into the body can be improved without compromising the flexibility of the distal end part 7 of the catheter tube 5.

### Second Embodiment

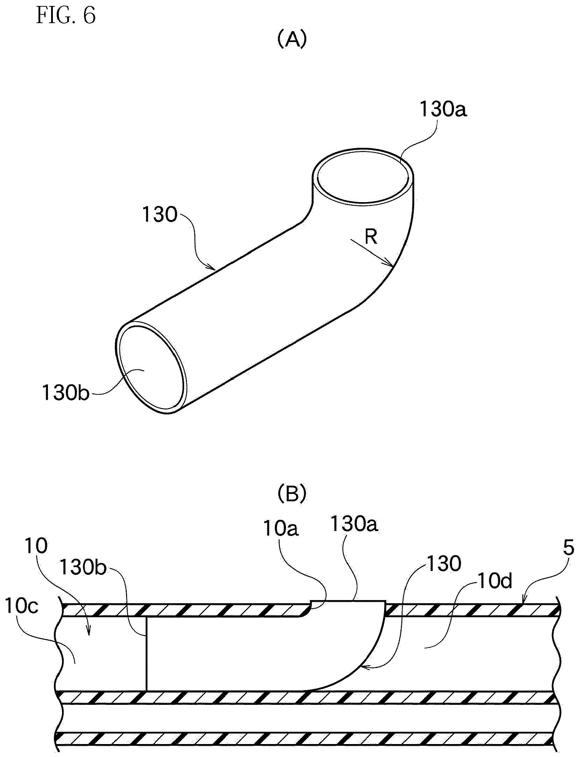

The second embodiment of the present invention has the same structure and effect as the aforementioned first embodiment except that the tube member 130 shown in Fig.6(A) and Fig.6(B) is used in place of the tube member 30 of the above mentioned first embodiment. The part which is different from the first embodiment will only be explained in below.

This tube member 130 is different from the tube member 30, and it is a tubular molded article which is formed into a curved shape in advance so that it is curved having a predetermined curvature R towards the proximal end opening 130a. The curvature R is determined so that the guide wire 20 can easily pass between the proximal end opening 130a and the distal end opening 130b at the inside of the tube member 130. As shown in Fig.6(B), the proximal end opening 130a of the tube member 130 is integrally formed by bonding with proximal side wire insertion opening 10a formed in the middle of the catheter tube 5 using the adhesive agent or so. The distal end opening 130b of the tube member 130 is inserted towards the distal side main lumen 10c, and the outer peripheral face of the distal end opening 130b is closely attached to the inner peripheral face of the distal side main lumen 10c.

### Third Embodiment

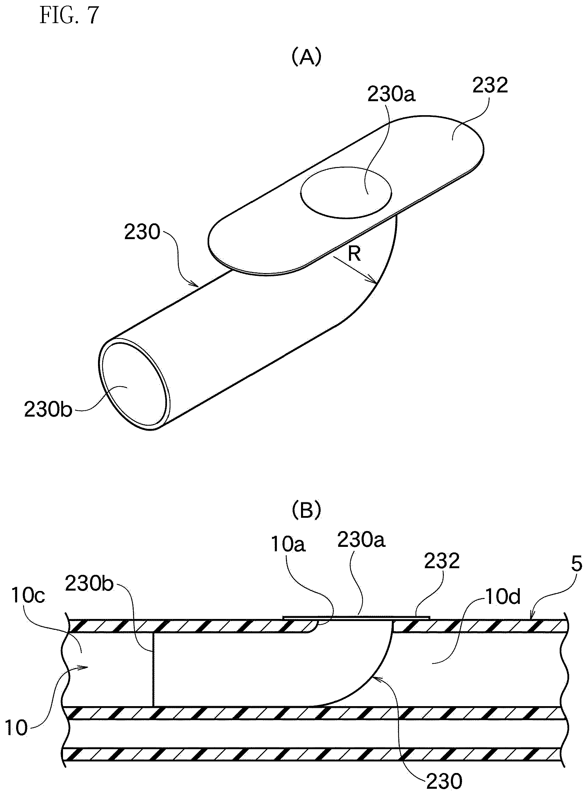

The third embodiment of the present invention has the same effect and structure as the aforementioned first and second embodiments, except that the tube member 230 shown in Fig.7 (A) and Fig.7(B) is used in place of the tube member 130 of the above mentioned second embodiment. The part which is different from the first and second embodiments will only be explained in below.

This tube member 230 is different from the tube member 130, and at the proximal end opening 230a of the tube member 230, the flange part 232 is integrally formed, and this flange 232 is bonded to the outer peripheral face of the catheter tube 5 positioned around the proximal side wire insertion opening 10a. By taking such constitution, the process of integrally bonding the periphery of the proximal end opening 230a of the tube member 230 with the proximal side wire insertion opening 10a can be done easily. Also, the bonding strength thereof improves as well. Note that, as shown in Fig.7(B), the distal end opening 230b of the tube member 230 is inserted towards the distal side main lumen 10c, and the outer peripheral face of the distal end opening 230b closely attaches with the inner peripheral face of the distal side main lumen 10c.

Note that, the present invention is not limited to the aforementioned embodiments, and it can be modified variously within the scope of the present invention.

For example, in the above embodiment, the balloon catheter 1 for removing the calculus comprises one balloon; however it may be plurality of balloons. Also, the catheter tube 5 does not necessarily have to have the contrast agent lumen 9, and other lumen having the function other than the above mentioned functions can be formed. Also, the contrast agent lumen 9 may have a function other than sending the contrast agent to the exhaustion opening 12. For example, a fluid such as a saline solution may be flushed into the contrast agent lumen 9, and the fluid can be discharged from the exhaustion opening 12, thereby it may function to wash out the calculus or by said fluid. In this case, the exhaustion opening 12 is tilted against the wall of the catheter tube 5 so that the fluid is discharged by tilting towards the proximal end direction side with respect to the center axis of the catheter.

Also, in the above mentioned embodiment, the outer diameter of the distal end part 7 of the catheter tube 5 is made smaller than the proximal end part, but it is not necessarily limited thereto, and for example the outer diameter of the distal end part and the proximal end part may be substantially the same.

Also, in the above mentioned embodiment, the inflating part 3 of the balloon 2 is inflated to be approximately rotationally symmetrical taking the center axis of the catheter tube 5 as the symmetrical axis; however as described in JP Patent Application Laid Open No.2008-194166, an eccentric inflating means may be provided to at least part of the circumference direction of the balloon 2 so that the inflating part 3 inflates eccentrically with respect to the axis of the catheter tube 5.

Further, the balloon catheter 1 for removing the calculus may be those used for removing the calculus from the body, and it is not limited to the removal of the gall stone. Also, the treating part of the treatment instrument for endoscope of the present invention may be other than the balloon 2, and it may be the balloon catheter used for other than calculus removal, or the treatment instrument for endoscope other than balloon catheter.

### Fourth Embodiment

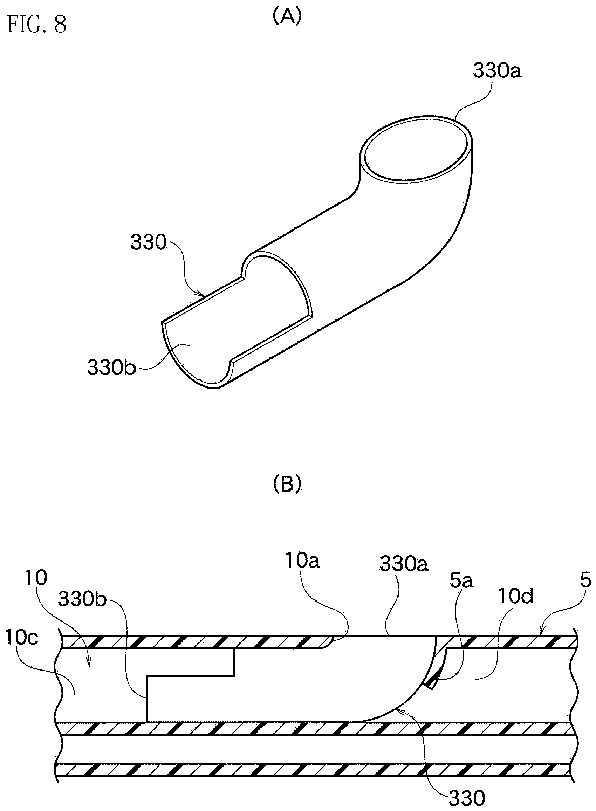

The fourth embodiment of the present invention has the same constitution and effect as the aforementioned first embodiment, except that the tube member 330 shown in Fig.8(A) and Fig.8(B) is used in place of the tube member 30 of the above mentioned first embodiment, and the supporting part 5a is provided at the proximal side wire insertion opening 10a of the catheter tube 5 as shown in Fig.8(B) and Fig.9. Hereinafter, only the difference will be described.

The tube member 330 shown in Fig.8(A) and Fig.8(B) is different from the tube member 30, and the distal end part has a transverse cross section of approximately "U" shape, that is it has a shape extending from the distal end opening 330b to the proximal end side, such as a gutter shape. Such tube member 330 is produced by removing part of the distal end side of the tube used for constituting the tube member 330. Also, the tube member 330 is different from the tube member 30, and the proximal end opening 330a is oval shape wherein the length along the axial direction of the catheter tube 5 is a long diameter. Such tube member 330 is produced for example by cutting the proximal end side of the tube for constituting the tube member 330 diagonally with respect to the axial direction of the tube. As shown in Fig.8(B), the proximal end opening 330a of the tube member 330 is integrally formed by bonding with the supporting part 5a and the proximal side wire insertion opening 10a formed in the middle of the catheter tube 5 using the adhesive agent.

As shown in Fig.9, the supporting part 5a is integral with the catheter tube 5, and it has a depressed shape wherein the peripheral wall of the catheter tube 5 adjacent to the proximal end side of the opening edge of the proximal side wire insertion opening 10a is pressed from outside to inside. The supporting part 5a is, for example, provided as a part of the catheter tube 5 by forming the proximal side wire insertion opening 10a to the catheter tube 5, then the heated soldering iron is pressed against the peripheral wall of the catheter tube 5 adjacent to the proximal end side of the proximal side wire insertion opening 10a to form a depressed shape as shown in Fig.9. In the fourth embodiment, by using the tube member 330 having the gutter shaped distal end part, the step difference between the distal end opening 330b and the distal side main lumen 10c of the tube member 330 caused by the thickness of the tube member 330 will be present throughout the entire circumference of the distal side main lumen 10c, thereby the guide wire can be prevented from easily hooking to this step difference. Also, the supporting part 5a can improve the bonding strength of the tube member 330 against the catheter tube 5. Also, by supporting this along the curved part of the tube member 330, the curved part of the tube member 30 can be held at the appropriate angle.

### NUMERICAL REFERENCES

1...Balloon catheter for removing calculus (treatment instrument for endoscope)
2...Balloon (treating part)
3...Inflating part
4...Bonding part
5...Catheter tube (multi-lumen tube)
6...Proximal end part
7...Distal end part
8...Balloon lumen
9...Contrast agent lumen
10...Main lumen
10a...Proximal side wire insertion opening
10b...Distal side wire insertion opening
10c...Distal side main lumen
10d...Proximal side main lumen
11...Fluid discharge opening
12...Exhaustion opening
13...Cover
14a to 14c...Branch pipe
15a to 15c...Hub
20...Guide wire
30, 130, 230, 330...Tube member
30a, 130a, 230a, 330a...Proximal end opening
30b, 130b, 230b, 330b...Distal end opening

## Claims

1. A treatment instrument (1) for an endoscope, comprising:
a multi-lumen tube (5) formed with multiple lumens including a main lumen (10) along longitudinal direction and formed with a distal side wire insertion opening (10b) connecting to said main lumen (10) at the distal end, and
a treating part (2) provided at a distal end part (7) of said multi-lumen tube (5),
a proximal side wire insertion opening (10a) connecting to said main lumen (10) is formed in a middle of the longitudinal direction of said multi-lumen tube (5) at a proximal side than said treating part (2), **characterised in that**
a tube member (30) is inserted towards the distal end direction of said main lumen (10) from said proximal side wire insertion opening (10a), and
a peripheral of a proximal end opening (30a) of said tube member (30) is bonded to a peripheral of said proximal side wire insertion opening (10a), wherein
an outer peripheral face of the distal end opening (30b) of said tube member (30) is adhered to the inner circumference face of said main lumen (10).

2. The treatment instrument for the endoscope as set forth in claim 1, wherein a stylet can be inserted in a removable manner at said main lumen (10) positioned to more proximal side than the proximal end of said tube member (30).

3. The treatment instrument for the endoscope as set forth in claim 1 or 2, wherein a flange part (232) is integrally formed at the proximal end of said tube member (30), and said flange part (232) is bonded to an outer peripheral face of said multi-lumen tube (5) which is positioned around said proximal side wire insertion opening (10a).

4. The treatment instrument for the endoscope as set forth in any one of claims 1 to 3, wherein a balloon (2) is attached to the distal end part (7) of said multi-lumen tube (5) at said treating part (2), and
an inside of said balloon (2) is connected to a balloon lumen (8) which is one of the lumen among said multi-lumen tube (5).

5. The treatment instrument for the endoscope as set forth in any one of claims 1 to 4, wherein
a length of the tube member (30) is about the length wherein the distal end of said tube member (30) does not project out from said distal side wire insertion opening (10b).

6. The treatment instrument for the endoscope as set forth in any one of claims 1 to 5, wherein
at the distal end part (7) of said multi-lumen tube (5), a small diameter part is formed which has an outer diameter smaller than other part of said multi-lumen tube (5), and said small diameter part being equipped with said treating part (2), and
said proximal side wire insertion opening (10a) is formed to said multi-lumen tube (5) of the proximal side than said small diameter part.

7. The treatment instrument for the endoscope as set forth in any one of claims 1 to 6, wherein
the distal end part (7) of said tube member (30) has a gutter shape.

8. The treatment instrument for the endoscope as set forth in any one of claims 1 to 7, wherein
said multi-lumen tube (5) has a supporting piece (5a) for supporting said tube member (30), and
said supporting piece forming a depressed part adjacent to the proximal end side of an opening edge of said proximal side wire insertion opening (10a).

## Patentansprüche

1. Ein Behandlungsinstrument (1) für ein Endoskop, umfassend:
ein mehrlumiges Rohr (5), der mit mehreren Lumen ausgebildet ist, einschließlich eines Hauptlumens (10) entlang der Längsrichtung, und der mit einer distalseitigen Drahteinführungsöffnung (10b) ausgebildet ist, die mit dem Hauptlumen (10) am distalen Ende verbunden ist, und
einen Behandlungsteil (2), der an einem distalen Endteil (7) des mehrlumigen Schlauchs (5) vorgesehen ist,
eine proximalseitige Drahteinführungsöffnung (10a), die mit dem Hauptlumen (10) verbunden ist, in der Mitte der Längsrichtung des mehrlumigen Rohrs (5) auf einer proximalen Seite als der Behandlungsteil (2) ausgebildet ist, **dadurch gekennzeichnet, dass**
ein Rohrelement (30) von der proximalen Drahteinführungsöffnung (10a) in Richtung des distalen Endes des Hauptlumens (10) eingeführt wird, und
eine Peripherie einer proximalen Endöffnung (30a) des Röhrenelements (30) mit einer Peripherie der proximalseitige Drahteinführungsöffnung (10a) verbunden ist, wobei
eine äußere Umfangsfläche der distalen Endöffnung (30b) des Röhrenelements (30) an der inneren Umfangsfläche des Hauptlumens (10) angeklebt ist.

2. Das Behandlungsinstrument für das Endoskop nach Anspruch 1, bei dem ein Stilett abnehmbar in das Hauptlumen (10) eingeführt werden kann, das an der proximaleren Seite als das proximale Ende des Rohrelements (30) angeordnet ist.

3. Das Behandlungsinstrument für das Endoskop nach Anspruch 1 oder 2, wobei ein Flanschteil (232) einstückig an dem proximalen Ende des Röhrenelements (30) ausgebildet ist und der Flanschteil (232) mit einer äußeren Umfangsfläche des mehrlumigen Rohrs (5) verbunden ist, das um die proximale Drahteinführungsöffnung (10a) herum angeordnet ist.

4. Das Behandlungsinstrument für das Endoskop nach einem der Ansprüche 1 bis 3, wobei ein Ballon (2) an dem distalen Endteil (7) des mehrlumigen Rohrs (5) an dem Behandlungsteil (2) angebracht ist, und
eine Innenseite des Ballons (2) mit einem Ballonlumen (8) verbunden ist, das eines der Lumen des mehrlumigen Rohrs (5) ist.

5. Das Behandlungsinstrument für das Endoskop nach einem der Ansprüche 1 bis 4, wobei
eine Länge des Röhrenelements (30) etwa die Länge ist, bei der das distale Ende des Röhrenelements (30) nicht aus der distalseitigen Drahteinführungsöffnung (10b) herausragt.

6. Das Behandlungsinstrument für das Endoskop nach einem der Ansprüche 1 bis 5, wobei
an dem distalen Endteil (7) des mehrlumigen Rohrs (5) ein Teil mit kleinem Durchmesser ausgebildet ist, der einen kleineren Außendurchmesser als der andere Teil des mehrlumigen Rohrs (5) hat, und der Teil mit kleinem Durchmesser mit dem Behandlungsteil (2) ausgestattet ist, und
die proximalseitige Drahteinführungsöffnung (10a) an dem mehrlumigen Rohr (5) der proximalen Seite als der Teil mit dem kleinen Durchmesser ausgebildet ist.

7. Das Behandlungsinstrument für das Endoskop nach einem der Ansprüche 1 bis 6, wobei
der distale Endteil (7) des Röhrenelements (30) eine Rinnenform aufweist.

8. Das Behandlungsinstrument für das Endoskop nach einem der Ansprüche 1 bis 7, wobei
der mehrlumige Rohr (5) ein Stützteil (5a) zum Stützen des Rohrelements (30) aufweist, und
wobei das Stützteil einen vertieften Teil angrenzend an die proximale Endseite einer Öffnungskante der proximalen Drahteinführungsöffnung (10a) bildet.

## Revendications

1. Instrument de traitement (1) pour un endoscope, comprenant :
un tube à plusieurs lumières (5) formé avec plusieurs lumières comprenant une lumière principale (10) le long de la direction longitudinale et formée avec une ouverture d'insertion de fil du côté distale (10b) se raccordant à ladite lumière principale (10) au niveau de l'extrémité distale, et
une partie de traitement (2) prévue au niveau d'une partie d'extrémité distale (7) dudit tube à plusieurs lumières (5),
une ouverture d'insertion de fil du côté proximal (10a) se raccordant à ladite lumière principale (10) est formée dans une partie centrale de la direction longitudinale dudit tube à plusieurs lumières (5) au niveau d'un côté proximal par rapport à ladite partie de traitement (2), **caractérisé en ce que** :
un élément de tube (30) est inséré vers la direction d'extrémité distale de ladite lumière principale (10) par ladite ouverture d'insertion de fil du côté proximal (10a), et
une périphérie d'une ouverture d'extrémité proximale (30a) dudit élément de tube (30) est reliée à une périphérie de ladite ouverture d'insertion de fil du côté proximal (10a), dans lequel :
une face périphérique externe de l'ouverture d'extrémité distale (30b) dudit élément de tube (30) est fixée sur la face circonférentielle interne de ladite lumière principale (10).

2. Instrument de traitement pour un endoscope selon la revendication 1, dans lequel un stylet peut être inséré d'une manière amovible au niveau de ladite lumière principale (10) positionnée du côté plus proximal que l'extrémité proximale dudit élément de tube (30).

3. Instrument de traitement pour un endoscope selon la revendication 1 ou 2, dans lequel une partie de bride (232) est formée, de manière solidaire, au niveau de l'extrémité proximale dudit élément de tube (30), et ladite partie de bride (232) est reliée à une face périphérique externe dudit tube à plusieurs lumières (5) qui est positionnée autour de ladite ouverture d'insertion de fil du côté proximal (10a).

4. Instrument de traitement pour un endoscope selon l'une quelconque des revendications 1 à 3, dans lequel un ballonnet (2) est fixé à la partie d'extrémité distale (7) dudit tube à plusieurs lumières (5) au niveau de ladite partie de traitement (2), et
un intérieur dudit ballonnet (2) est raccordé à une lumière de ballonnet (8) qui est l'une des lumières parmi ledit tube à plusieurs lumières (5).

5. Instrument de traitement pour un endoscope selon l'une quelconque des revendications 1 à 4, dans lequel :
une longueur de l'élément de tube (30) représente environ la longueur dans laquelle l'extrémité distale dudit élément de tube (30) ne fait pas saillie de ladite ouverture d'insertion de fil du côté distal (10b).

6. Instrument de traitement pour un endoscope selon l'une quelconque des revendications 1 à 5, dans lequel :
au niveau de la partie d'extrémité distale (7) dudit tube à plusieurs lumières (5), on forme une partie de petit diamètre qui a un diamètre externe inférieur à l'autre partie dudit tube à plusieurs lumières (5), et ladite partie de petit diamètre étant équipée avec ladite partie de traitement (2), et
ladite ouverture d'insertion de fil du côté proximal (10a) est formée sur ledit tube à plusieurs lumières (5) du côté proximal que ladite partie de petit diamètre.

7. Instrument de traitement pour un endoscope selon l'une quelconque des revendications 1 à 6, dans lequel :
la partie d'extrémité distale (7) dudit élément de tube (30) a une forme de gouttière.

8. Instrument de traitement pour un endoscope selon l'une quelconque des revendications 1 à 7, dans lequel :
ledit tube à plusieurs lumières (5) a une pièce de support (5a) pour supporter ledit élément de tube (30), et
ladite pièce de support formant une partie enfoncée adjacente au côté d'extrémité proximal d'un bord d'ouverture de ladite ouverture d'insertion de fil du côté proximal (10a).
